# EUROPEAN PATENT APPLICATION

(11) **EP 2 774 610 A1**
(43) Date of publication of application: **10.09.2014**
(21) Application number: 14157882.3
(22) Date of filing: 05.03.2014
(51) Int. Cl.: A61K 31/197, A61K 31/409, A61K 41/00

(54) **Photosensitizer for use as Virus-inactivating medicaments**

(30) Priority: 07.03.2013 CN 201310072176
(71) Applicant: Beijing Ecyber Medical Technology R & D Center Co., Ltd., Beijing 100176 (CN)
(72) Inventor: SUN, Keke, Beijing 100176 (CN); SUN, Qiying, Beijing 100176 (CN)
(74) Representative: Von Kreisler Selting Werner - Partnerschaft von Patentanwälten und Rechtsanwälten mbB

(57) **Abstract**

The invention relates to the field of medicament use, in particular to the clinical use of photosensitizers for inactivating viruses in the treatment of blood vessel plaque, leukemia, AIDS, hepatitis and the like. The invention is based on the affinity of photosensitizer to blood vessel plaque focus, leukemia cells, tumor, and/or virus, which is several times higher than that to normal tissues, to destroy focus cells or virus by generating singlet oxygen (¹O₂) under the effect of a method using laser, X-ray, ultrasound and medicament. The effects for treating the above diseases are achieved without serious side effects to the normal human tissues and organs.

## Description

### Technical Field

The invention relates to the field of medicaments, and particularly to the use of photosensitizers in inactivating viruses for treating diseases.

### Background Art

Cardiovascular and cerebrovascular diseases are one of the main causes leading to death throughout the world, and cause 15 million deaths every year. In Western countries, atherosclerosis is a main cause of cardiovascular and cerebrovascular diseases.

Cardiovascular and cerebrovascular diseases are the main cause of morbidity or death. The cause of cardiovascular and cerebrovascular diseases is that plaque formed in artery over time reduces the blood flow to the specific organs including brain and cardiac muscle. In certain cases, the reduction of blood flow causes symptoms of transient ischemic attack, scelalgia or angina. If arterial obstruction becomes more serious, it may lead to the injury of brain, leg or cardiac muscle, and the injury is fatal.

A method for treating cardiovascular and cerebrovascular diseases and avoiding further tissue injury is carried out by invasively eliminating plaque, which is generally achieved by invasive surgery. Another alternative method is achieved by balloon angioplasty, which includes inserting blood vessel with a catheter. During the process, artery stents may also be implanted. When angioplasty treatment may not be used due to the type of the plaque, the plaque may be bypassed through grafting a new blood vessel around the plaque region during the vascular surgery or cardiosurgery. Angioplasty or bypass surgery may not be available for some patients, for example, the patients may not receive such treatment due to advanced age or poor health, or no one of the above treatments is suitable for the plaque. In such case, the patients must try to control the disease by medical management, such as medications. Since the surgery treatment for arterial plaque is invasive, and has risk of complication, it is not suitable for all patients. Therefore, a minimally invasive method is required for reducing or eliminating the formation of plaque in artery. The US Patents with patent No. 5657760, patent No 5590657 and patent No 5524620 have disclosed non-invasive methods for treating the redundant substances in tissues and blood vessels, especially cardiac vessels. However, these methods are not suitable for to reduce the plaque, still less the use in blood vascular system.

Radiotherapy and chemotherapy have certain therapeutic effect on leukemia. However, most of the therapies adopting traditional chemotherapy have unsatisfied therapeutic effect on most leukemia, except on M3 leukemia.

In comparison with common chemotherapy, photodynamic therapy (PDT) is a new therapy developing recently, and may damage malignant cells by bio-photosensitization. Its principle is that some photosensitizers having high fluorescent quantum yield are injected into body, and the photosensitizers have affinity to leukemia cells higher than that to normal cells, so to retain in leukemia cells. Once irradiated with strong light, the photosensitizers absorb photons and jump to an excited state, and then transfer the energy to the surrounding oxygen molecules, to generate singlet oxygen. Singlet oxygen is a highly toxic agent for cells and has acting path including direct damage, that is, the singlet oxygen destroys the PDT sensitive cellular substructures, such as cell membrane, mitochondrion or liposome, to cause the damage of the cells.

In comparison with traditional chemotherapy, PDT has the advantages of relative selection specificity, low toxic and side effect, no invasion, and no accumulative toxicity. Recent years, departments of health in many countries like America, Japan, Holland, and Canada have successively confirmed the legality of the new tumor therapy. At the same time, the PDT therapy has also received more attention in non-tumor therapy, and is used for removing harmful cells or tissues. PDT utilizes photochemical reaction and a series of physiological immune reaction triggered by the photochemical reaction to achieve the aim for treating malignant tumors, and has many important advantages: (1) good selectivity: photodynamic therapy performs the therapy by photochemical reaction excited by illumination, is effective only in the region of illumination, and thus has almost no damage to normal tissues and cells outside the region of illumination; (2) low toxic and side effects: the photodynamic medicaments which enter into the tissues will trigger the phototoxic reaction to kill the tumor cells only when reaching a certain concentration and receiving sufficient light irradiation, no phototoxic reaction is triggered in the non-light irradiated part of the human body, thus the hematopoietic function, the immune function and the organ function in vivo are no affected, and in other words, photodynamic therapy has no severe toxicity as radiotherapy and chemotherapy; (3) minimal invasion: laser may be introduced to pathological tissue by means of optical fibers, endoscopes, ultrasonographies and other interventional technologies for therapy, so as to avoid injuries and pains caused by thoracotomy and laparotomy; (4) synergy with other therapies; and (5) repeatable treatment: since photosensitizer has no toxicity, and tumor cells have no drug tolerance to photosensitizer, the photodynamic therapy may be used repeatedly.

The fundamental principle of photodynamic therapy is that: the photosensitizer selectively aggregates around the tumor tissues and is excited to the excited state under excitation of light with a specific wavelength; the photosensitizer in the excited state may excite the dissolved oxygen in the tissues to form singlet oxygen with high oxidizing property and reactive oxygen species (ROS) in other forms; and the reactive oxygen species has toxicity and may destroy biomacromolecules such as proteins and nucleic acids, so as to cause necrosis of tumor cells. Currently, tens of thousands of patients all over the world have received the treatment of the therapy. The photodynamic therapy may treat dozens of cancers, including esophageal carcinoma, lung cancer, cerebroma, head and neck cancer, ocular tumor, pharyngeal cancer, tumor of chest wall, breast carcinoma, mesothelioma of pleura, abdominal sarcoma, bladder carcinoma, gynecological tumor, rectal cancer, cutaneous cancer and the like.

In the process of developing the photodynamic therapy, the selection and the use of photosensitizer is the core element. Photosensitizer for the photodynamic therapy should meet the following conditions: (1) maximum absorption wavelength between 600-800 nm, and absorption between 400-600 nm as small as possible; (2) high singlet oxygen yield; (3) strong phototoxicity and weak dark toxicity; (4) high retention ratio in malignant tumor tissues; (5) single component; (6) water-solubility; and (7) fluorescence.

Photodynamic therapy utilizes the photochemical reaction of the photosensitizer (PS) excited by laser, and the reaction product such as singlet oxygen, free radical and the like may kill cells. Different from three main traditional tumor treatment means including surgery, radiotherapy and chemotherapy, the photodynamic therapy has the advantages of selectivity, irreversible destruction of diseased tissues and no damage on peripheral normal cells. Application of the photodynamic therapy (PDT) in treatment of malignant tumors, especially superficial tumors (such as esophageal cancer, bladder carcinoma, oral squamous cell carcinoma and malignant melanoma) is increasingly becoming the hot spot of clinical research in recent years. However, the photosensitizers used in current PDT therapy have many disadvantages, such as long retention time in skin, slow excretion, easy cause of side effect, long light exposure avoiding time, poor selectivity and the like.

A photooxidation dynamic therapy is applied for treating tumors, and is characterized in that photosensitizer is irradiated with laser to generate oxygen free radical in tumor focus region, to kill tumor cells. The method has the advantages of high safety, less side effects, repeatable treatments, good compliance, and wide anti-tumor spectrum, suitability for systemic treatments, and excellent therapeutic effect for many malignant tumors, especially for malignant tumors with superficial foci.

However, in application of the photooxidation dynamic therapy for treating tumor, the first generation of photosensitizer is generally haematoporphyrins, which have insensitivity to photoresponse, low targeting property and high phototoxicity, and requires dark treatment after administration causing great inconvenience for life, and interventional administration causing injury to the patients. The second generation of photosensitizer is generally porphin or its derivatives, which has the main features of long response wavelength, low phototoxicity, no requirement for avoiding light exposure after administration, administration by intravenous injection, and small injury to patients, and disadvantages of insensitivity to photoresponse leading to unsatisfied killing effect on tumor cells, easy photodegradation, difficult storage and requirement for preparation before use.

For example, a mucosal drug delivery system for photodynamic diagnosis and therapy is disclosed in Chinese patent with publication No. CN1435261A, consists of gelling material of thermosetting agent, penetration enhancer and cellulose compounds, which is liquid at low temperature, and transforms to gel state at body temperature after being administered. Gelling agent refers to thick liquid or semi-solid preparation in the forms of solution, suspension, or emulsion prepared from medicaments and adjuvants which may form gel, and is suitable for topical application at skin or body cavity, such as nasal cavity, vagina and rectum. Gelling agent matrixes are divided into aqueous and oilymatrixes, and the aqueous gelling agent matrixes generally consists of water, glycerol, or propylene glycol and cellulose derivatives, carbomer and alginates, gum tragacanth, gelatin, starch and the like.

A hydrophilic in situ gelling preparation precursor capable of rapidly dissolution is disclosed in US patent US20080069857, and includes two different polysaccharide materials, such as derivates of HA, and cellulose compounds. However, the two different polysaccharide materials are preserved separately, and mixed in application for rapid cross-linking in water so as to form gel.

Photosensitizer kills tumor cells through above mentioned mechanisms, but has less injury to normal tissues. Comparing with the three traditional tumor treatment means including surgery, chemotherapy and radiotherapy, photodynamic therapy has good application prospect in treatment of tumor as photodynamic therapy has dual selectivity, i.e. directional irradiation effect of optical fibers and retention effect of the medicament in tumor tissues.

With the development of the research, photodynamic therapy is found to realize selective damage to target tissues by the reactive oxygenspecies (ROS) generated in photodynamic reaction triggered by the combination of photosensitizer medicament, irradiation, and oxygen molecule in tissues. During the process of PDT, the photosensitizer absorbs light energy, and then transforms from a ground state to a triplet excited state having relatively long duration after experiencing a transient single excited state. The photosensitizer in the excited state may cause two types of photodynamic reactions. In one photodynamic reaction, the photosensitizer in the triplet excited state may directly react with cell membranes or some biomacromolecules, transfer a hydrogen atom (electron) to form free radical, which interacts with the tissue oxygen to generate ROS (type I reaction) capable of killing target cells. In the other photodynamic reaction, the photosensitizer in the triplet excited state also may directly transfer the energy to the oxygen molecule, to form an effective ROS, i.e. singlet oxygen, to kill target cells (type II reaction). Type I reaction and type II reaction occur simultaneously, the ratio of them depends on the type of the photosensitizer, the concentration of the substrate and tissue oxygen, as well as the binging tightness of the photosensitizer with the substrate. In the whole process, the photosensitizer transforms to the excited state from the ground state, then returns back to the ground state, and acts as a catalyst. Sufficient concentration of the tissue oxygen is required for PDT, and the photosensitization may not occur in oxygen-deficient tissue regions. The density of photodynamic effect is influenced by oxygen content in tissues; for example, the photodynamic effect is reduced in the oxygen-deficient regions of the tumor tissues. In the photodynamic process, after the photosensitizer is irradiated, the oxygen pressure in the tissues reduces rapidly and significantly. The reduction of the tissue oxygen limits the photosensitivity reaction, thereby influencing the killing effect of PDT. As it may be seen, the content of the oxygen in the tissues, as one of the three main photodynamic factors, may not be ignored in practical application. In order to satisfy the requirement of the photodynamic action for oxygen, PDT adopting stepwise irradiation is performed by some scholars, and reoxidation of the tissues is allowed, so as to overcome the problem. Some scholars use HBO to increase the oxygen content in the tissues, as the adjuvant therapy of the photodynamic therapy, so as to increase the lethal effect on tumor cells directly and indirectly. Chinese Patent Laser Irradiation Device With Hyperbaric Oxygen Chamber (Patent No. CN85103131) a device performing photodynamic therapy under hyperbaric oxygen environment for increasing oxygen content in tissues, to improve the effect of the photodynamic therapy. Chinese Patent Assembly of Photodynamic Therapy (Application No. 03104558.8 and Publication No. CN1438048A) discloses a device for increasing the concentration of tissue oxygen in the target site by using a transdermal negative pressure drug delivery therapy apparatus to improve the effect of the photodynamic therapy. New safe and efficient photosensitizers and new methods for increasing the oxygen content in target tissues have been sought in order to further increase oxygen content in target tissues and improve therapeutic effect of photodynamic therapy.

Traditional photodynamic therapy (PDT) utilizes the affinity of the photosensitizer to tumor, leukemia cells, blood vessel plaque, dermatosis and viruses, which is several times higher than that to the normal tissues, and then irradiates focus, cells or viruses with visible light having specific wavelength capable of being absorbed by the photosensitizer; after absorbing the light energy, the photosensitizer in the focus transforms O₂ to ¹O₂, which then causes apoptosis and/or necrosis of pathological cells, so as to achieve therapeutic effect. However, due to poor tissue penetrability, difficult quantitative treatment and frequent lack of O₂ in tumors, the actual effect of clinical PDT is not very satisfactory; at the same time, in many cases, clinically, invasive and non-invasive intervention means are required in clinical to introduce light source into the pathological sites, which is hard for doctors and the patients to accept.

### Summary of the Invention

The present invention provides the following technical schemes to solve above mentioned problems.

Application of photosensitizer in preparation of virus inactivating medicaments for treating disease is characterized in that, by virtue of the affinity of photosensitizers to blood vessel plaque focus, leukemia cells, tumor, AIDS and hepatitis virus-induced neurological disease, which is several times higher than that to normal tissues, focus cells or virus are destroyed without side effects to the normal human tissues by generating sufficient amount of singlet oxygen, i.e. ¹O₂ with the endogenous and/or exogenous hydrogen peroxide injection as substrate and under the catalysis of a photosensitizer, which is activated by the effect of a method using laser, X-ray, ultrasound and medicament, so as to treat above diseases efficiently.

Said photosensitizer is acidified porphyrin, hemoporphyrin and the derivatives thereof, 5-ALA, etc.

The application includes the following steps:
step 1, determining treating target regions of focus by performing systemic CT plain scan on ill body or imaging method, or suffusing said disease with systemic irradiation;
step 2, determining treatment plan by plotting treating target regions of the focus on a treatment planning system or systemically irradiating the target regions, and irradiating once;
step 3, allowing oral administration of 5-ALA at 20-80 mg/kg 2-6 h before irradiation, slowly intravenously dripping carbamide peroxide at 80 mg/kg immediately before and after irradiation, irradiating focus once with 4-6 Gy immediately after dripping carbamide peroxide, and systemically irradiating once at 0.5-1.0 Gy;
step 4, performing the treatment course 4-6 times according to the different types of the diseases; and
step 5, reexamining with CT or other imaging methods in 5-7 days after one treatment course and recording regression status.

Treatment principle: treatment mechanism is that the affinity of photosensitizer to big foam cells in blood vessel plaque, leukemia cells and viruses is several times higher than that to normal tissues, the photosensitizer generates singlet oxygen under high energy ray excitation to destroy big foam cells, leukemia cells and viruses, so as to achieve the purpose for treating above diseases.

### Beneficial Effects

1. Good therapeutic effect: especially in destroying mitochondrion in abnormal proliferative cells, and irreversibly killing malignant cells; and
2. Low side effects: photosensitizer concentrates in the focus and is activated in the focus target region, to allow the reaction to be centralized at the focus region, leukemia cells and virus surface, and less reactions on normal cells and sites.

The invention utilizes the affinity of photosensitizer to blood vessel plaque focus, tumor, leukemia cells and viruses, which is several times higher than that to normal tissues, to destroy focus cells or viruses by generating singlet oxygen (¹O₂) under the excitation of X-ray, laser, ultrasound or medicament. There is no severe side effect on normal human tissues and organs.

### Description of Drawings

Figure 1: photosensitizer enters into human body and accumulates at blood vessel plaque;
Figure 2: wavelength of X-ray used; and

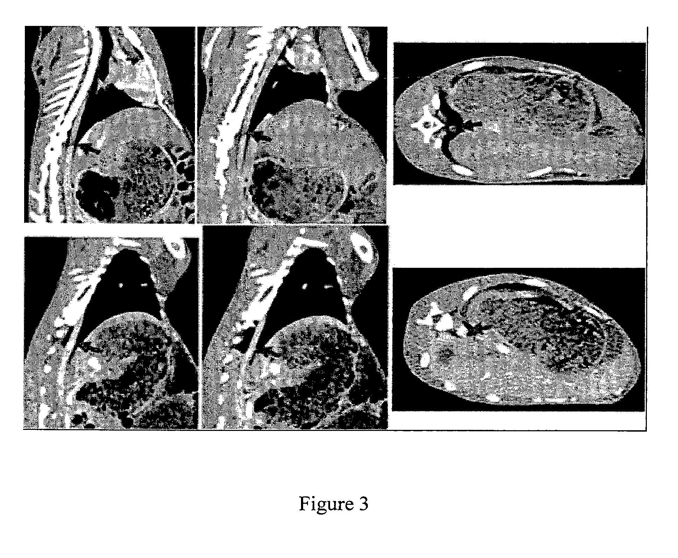

Figure 3: the presence of plaque in the blood vessel of a rabbit was confirmed by

CT examination, then the rabbit was given with the photosensitizer for oral administration and irradiated with high energy X-ray, CT examination was performed 5 days later, and it was found that the plaque is eliminated, and the therapeutic purposes was effectively achieved.

In the figures, 1 represents the plaque; and 2 represents the photosensitizer.

### Detailed Description of the Preferred Embodiments

### Embodiment 1: Treatment of blood vessel plaque

Virus inactivating application of photosensitizer for treating disease is characterized in that wherein by the affinity of photosensitizer to blood vessel plaque focus, leukemia cells, tumor, HIV or hepatitis viruses, which is several times higher than that to normal tissues, focus cells or viruses are destroyed by singlet oxygen generated under the effect of laser, X-ray, ultrasound or medicament method.

Wherein said photosensitizer is acidified porphyrin, hemoporphyrin and the derivatives thereof, or 5-ALA.
1. Experimental method:
   (1) Male New-Zealand Experimental rabbits each having body weight of 3-4 kg were fed with feed containing 1% cholesterol and lard for 11 weeks till the appearance of atherosclerotic plaque in bilateral fundus vessels and aorta.
   (2) Experimental rabbits were anesthetized with ketamine and thiopental sodium.
   (3) Treating target region of aorta plaque was determined with systemic CT plain scan and CTA enhancement.
   (4) Treatment plan was determined by plotting treating target region of aorta plaque on a treatment planning system, once irradiation was performed, and normal feeding was recovered.
   (5) 5-ALA (80 mg/kg in saline 5 mL) was intraperitoneally injected 2 h before irradiation, carbamide peroxide (injection containing H₂O₂ for, 80 mg/kg) in saline 5mL was injected slowly at auricular vein immediately before irradiation, and the treatment plan was performed immediately after the injection of carbamide peroxide.
   (6) Reexamination with CTA was performed 5-7 days after irradiation, and the regression of blood vessel plaque was recorded.

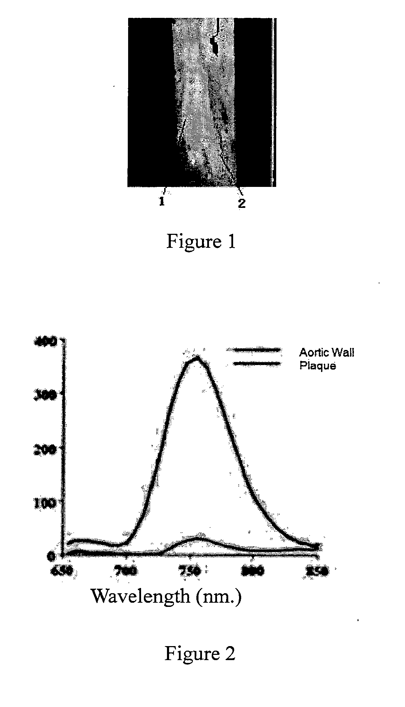

The New-Zealand rabbit model of blood vessel plaque is a common animal experimental model. New-Zealand rabbit was fed with high fat and high cholesterol for three months, and the formation of thoracic aorta blood vessel plaque was shown by examination with CTA angiography and MR. The blood vessel plaque was taken as the target region and positioned, and a radiotherapy plan was made. 1.5 h after intraperitoneal injection of 5-ALA (80 mg/kg body weight), the substrate was intravenously infused, and the irradiation to the blood vessel plaque was performed once with 5 Gy. The New-Zealand rabbit was fed for 1 week. On the seventh day of the treatment, examination with CTA angiography and MR detection was performed again, and the result shows that the thoracic aorta blood vessel plaque completely disappeared (Shown in Fig. 2).

Finally, it should be noted that, obviously, the foregoing embodiment is only an example intended to clearly illustrate the application, rather than limit the embodiments. For an ordinary skill in the field, other changes in different forms or variations may be made on the basis of above description. There is no need and it is impossible to exhaustively list all embodiments. And obvious changes and variations extended herein are still in the protection scope of the present application. The use for treating leukemia and viral diseases is also in the protection scope of the present application.

## Claims

1. Use of a photosensitizer in the preparation of virus-inactivating medicaments for treating diseases, **characterized in that**, by virtue of the affinity of photosensitizers to blood vessel plaque focus, leukemia cells, tumor, AIDS and hepatitis virus-induced neurological disease, which is several times higher than that to normal tissues, focus cells or virus are destroyed without side effects to the normal human tissues by generating sufficient amount of singlet oxygen, i.e. ¹O₂ with the endogenous and/or exogenous hydrogen peroxide injection as substrate and under the catalysis of a photosensitizer, which is activated by the effect of a method using laser, X-ray, ultrasound and medicament, so as to treat above diseases efficiently.

2. The use according to the claim 1, **characterized in that** said photosensitizer is acidified porphyrin, hemoporphyrin and derivatives thereof, 5-ALA, and other photosensitizers.

3. The use according to the claim 1, **characterized in that** said use includes the following steps:
step 1, determining treating target regions of focus by performing systemic CT plain scan on ill body or imaging method, or suffusing said disease with systemic irradiation;
step 2, determining treatment plan by plotting treating target regions of the focus on a treatment planning system or systemically irradiating the target regions, and irradiating once;
step 3, allowing oral administration of 5-ALA at 20-80 mg/kg 2-6 h before irradiation, slowly intravenously dripping carbamide peroxide at 80 mg/kg immediately before and after irradiation, irradiating focus once with 4-6 Gy immediately after dripping carbamide peroxide, and systemically irradiating once at 0.5-1.0 Gy;
step 4, performing the treatment course 4-6 times according to the different types of the diseases; and
step 5, reexamining with CT or other imaging methods in 5-7 days after one treatment course and recording regression status.
